(19) Europäisches Patentamt
European Patent Office
Office européen des brevets

(11) Numéro de publication: **0 333 557 B1**

(12) **FASCICULE DE BREVET EUROPEEN**

(45) Date de publication de fascicule du brevet: **02.02.94**

(51) Int. Cl.$^5$: **C07C 311/16**

(21) Numéro de dépôt: **89400638.6**

(22) Date de dépôt: **07.03.89**

Le dossier contient des informations techniques présentées postérieurement au dépôt de la demande et ne figurant pas dans le présent fascicule.

# (54) Procédé de synthèse d'arylsulfonylalkylamide.

(30) Priorité: **17.03.88 FR 8803447**

(43) Date de publication de la demande:
**20.09.89 Bulletin 89/38**

(45) Mention de la délivrance du brevet:
**02.02.94 Bulletin 94/05**

(84) Etats contractants désignés:
**AT BE CH DE ES FR GB GR IT LI LU NL SE**

(56) Documents cités:
**EP-A- 0 007 623**
**EP-A- 0 027 906**

**METHODEN DER ORGANISCHEN CHEMIE, HOUBEN-WEYL, vol. IX, pages 609-610, 1955, Georg Thieme Verlag, Stuttgart, DE**

(73) Titulaire: **ELF ATOCHEM S.A.**
**4 & 8, Cours Michelet**
**La Défense 10**
**F-92800 Puteaux(FR)**

(72) Inventeur: **Commandeur, Raymond**
**Le Rocher**
**Avenue de Vénaria**
**F-38220 Vizille(FR)**
Inventeur: **Ghenassia, Elie**
**20 Avenue Albert 1er de Belgique**
**F-38100 Grenoble(FR)**

**EP 0 333 557 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

Il est important que ces plastifiants ne se dégradent pas avec la chaleur. L'incorporation du plastifiant se fait à des températures élevées (200 à 250 °C), c'est-à-dire que lors de sa mise en oeuvre, il ne doit pas être la source de formation de produite acides qui risquent de développer une coloration et d'autre part, nuire aux propriétés mécaniques des polymères (rupture de chaîne). La demande de brevet européen EP 7623 publiée le 6 Février 1980 décrit un procédé de purification d'arylsulfonylalkylamide par action d'un agent alcalin à 200 °C pour obtenir un produit thermostable. On a maintenant trouvé un procédé qui permet de synthétiser un arylsulfonylalkylamide thermostable et donc directement utilisable comme plastifiant dans les polyamides, sans avoir à utiliser un procédé de purification comme par exemple celui décrit dans EP 7623.

La présente invention concerne un procédé de synthèse d'arylsulfonylalkylamide de formule :

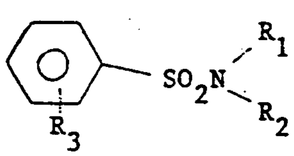

dans laquelle $R_1$ représente un atome d' hydrogène ou un groupe alkyle ayant de 1 à 10 atomes de carbone, $R_2$ représente un groupe alkyle ayant de 1 à 10 atomes de carbone et $R_3$ représente un ou plusieurs substituants identiques ou différents choisis dans le groupe constitué par les halogènes et les alkyles ayant jusqu'à 5 atomes de carbone, par réaction d'un arylsulfohalogénure et d'une alkylamine caractérisé en ce que :

a) on met en contact l'arylsulfohalogénure avec un excès d'alkylamine et une solution aqueuse d'un agent alcalin, la quantité d'agent alcalin étant en excès par rapport à l'arylsulfohalogénure engagé,

b) on élimine l'eau et l'alkylamine de la phase organique obtenue en a) par distillation,

c) du reste obtenu en b) on sépare l'arylsulfonylalkylamide.

Bien que $R_1$ et $R_2$ puissent être différents on utilise avantageusement les produits dans lesquels $R_1$ et $R_2$ sont identiques et parmi ces produits on préfère ceux dans lesquels $R_1$ et $R_2$ n'ont pas plus de 3 atomes de carbone. Une autre famille de produits intéressants est celle dans laquelle $R_1$ est un hydrogène et $R_2$ un alkyle ayant avantageusement de 2 à 6 carbones et de préférence 4.

Parmi les substituants du noyau benzénique on préfère le fluor, le chlore, le brome et le méthyle. Le noyau peut comporter plusieurs de ces substituants simultanément, c'est-à-dire qu'on peut avoir par exemple un méthyle et un ou plusieurs atomes de brome ou bien un méthyle et un ou plusieurs atomes de chlore. Des produits particulièrement intéressants sont ceux dans lesquels $R_3$ est de l'hydrogène, c'est-à-dire le noyau benzénique non substitué, $R_1$ est aussi de l'hydrogène et $R_2$ est un alkyle ayant de 2 à 6 atomes de carbone.

Parmi ces produits on utilise de préférence la N.n-butylbenzène sulfonamide de formule :

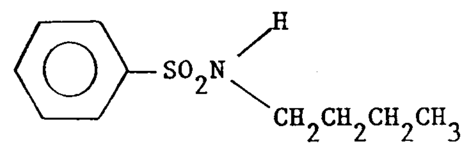

L'arylsulfohalogénure de départ est le produit de formule suivante :

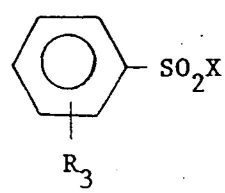

dans laquelle $R_3$ a la même signification que précédemment et X désigne un halogène. X désigne avantageusement le chlore et le brome et de préférence le chlore. L'alkylamine de départ est le produit de formule suivante :

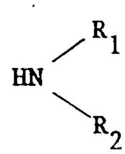

dans laquelle $R_1$ et $R_2$ ont la même signification que précédemment.

La réaction entre l'arylsulfohalogénure et l'alkylamine est essentiellement totale et nécessite en théorie une mole d'halogénure pour une mole d'amine, on obtient un mole d'HX qu'on transforme avec un agent alcalin.

On utilise de préférence le benzène sulfochlorure, c'est-à-dire le produit dans lequel $R_3$ est l'hydrogène et X le chlore et la n-butylamine c'est-à-dire le produit dans lequel $R_1$ est l'hydrogène et $R_2$ le n-butyl.

Dans l'étape a) il est essentiel d'utiliser un excès d'alkylamine, c'est-à-dire que pour une mole d'halogénure on utilise plus d'une mole d'amine.

Avantageusement cet excès en moles est de 20 %, c'est-à-dire 1,2 mole d'amine par mole d'halogénure engagée, et de préférence 5 à 15 %. On ne sortirait pas du cadre de l'invention en utilisant un large excès mais en fin de réaction il faudrait recycler d'importantes quantités d'amine.

Comme agent alcalin en solution aqueuse, on peut utiliser par exemple les hydroxydes, carbonates, bicarbonates et alcoolates des métaux alcalins et alcalins-terreux. On utilise avantageusement l'hydroxyde de sodium (soude) ou de potassium, et de préférence la soude. Bien que la concentration de la soude ou de la potasse soit sans importance, il est commode d'utiliser des solutions aqueuses entre 10 et 30 % en poids. La quantité d'agent alcalin nécessaire est fonction de la quantité de sulfohalogénure engagée en a), la stoechiométrie étant un équivalent alcalin pour une mole de sulfohalogénure, c'est-à-dire que si on utilise de la potasse ou de la soude il en faut au moins une mole pour une mole de sulfohalogénure. Il est nécessaire d'utiliser un excès d'agent alcalin par rapport à la quantité stoechiométrique qu'on vient de définir. On utilise avantageusement un excès molaire allant jusqu'à 10 % et de préférence compris entre 1 et 5 %. On ne sortirait pas du cadre de l'invention en utilisant un large excès de soude mais le procédé serait compliqué par les grandes quantités de produit excédentaire à éliminer.

On peut opérer en continu ou en discontinu et en ajoutant l'arylsulfohalogénure, l'alkylamine et la solution aqueuse d'agent alcalin dans un ordre quelconque ou pour partie dans un ordre, et pour partie dans un autre ordre. La seule condition à respecter est de ne pas détruire l'arylsulfohalogénure par réaction avec l'agent alcalin. Par exemple on peut mettre en contact l'arylsulfohalogénure avec l'alkylamine puis ajouter l'agent alcalin. On peut aussi couler l'arylsulfohalogénure dans un mélange agité d'agent alcalin en solution aqueuse et d'alkylamine. On emploie l'expression "mélange agité" parce que la solution alcaline et l'alkylamine ne sont généralement pas miscibles, par agitation on obtient une sortie d'émulsion instable. Selon une autre variante on peut aussi couler l'arylsulfohalogénure et la solution aqueuse d'agent alcalin dans l'alkylamine avec un léger retard pour la solution alcaline. Le "retard" s'apprécie en nombre de moles d'agent alcalin par rapport au nombre de moles d'arylsulfohalogénure.

Il est essentiel dans l'invention d'avoir en contact un arylsulfohalogénure, une alkylamine, de l'eau et un agent alcalin, on ne sortirait pas du cadre de l'invention en utilisant un agent alcalin anhydre et de l'eau ou un agent alcalin anhydre et une alkylamine en émulsion aqueuse. On peut utiliser un arylsulfohalogénure tel quel ou en solution dans un solvant, l'alkylamine peut aussi être telle quelle ou éventuellement dans un solvant, par exemple du toluène.

Bien que l'étape a) puisse se faire à toute température et pression pourvu bien sur que les produits ne soient pas décomposés, on préfère opérer à température ambiante ou proche de l'ambiante et à la pression atmosphérique ou une pression voisine, de telle sorte que l'halogénure soit liquide et que l'amine soit aussi liquide. Si ces conditions sont impossibles à réunir on se place dans une zone de pression et de température ou l'halogénure est liquide et l'amine est gazeuse. Ces conditions sont avantageusement une température inférieure à 150 °C et une pression inférieure à 5 bars relatifs.

On opère de préférence à la pression atmosphérique et à une température proche de la température ambiante, c'est-à-dire entre 0 et 50 °C.

La durée de cette étape a) est sans importance, mais la réaction est instantanée et sa durée est fixée par les conditions pratiques liées à l'appareillage et aux quantitées manipulées.

Cette durée est habituellement de l'ordre de 15 minutes à quelques heures.

Cette mise en contact est une opération connue en soi et peut se faire dans tout appareillage utilisé dans l'industrie chimique, on utilise avantageusement des appareils agités.

Quand tous les réactifs de cette étape a) ont été mis en contact on maintient avantageusement le milieu réactionnel sous agitation à une température comprise entre 20 et 100 °C et de préférence 40 et 70 pendant une période pouvant varier de quelques minutes à quelques heures et de préférence comprise entre une heure et trois heures. On procède ensuite à la séparation du milieu réactionnel obtenu en fin de l'étape a) en une phase aqueuse et une phase organique contenant essentiellement l'arylsulforylalkylamide, de l'alkylamine et quelques pourcents d'eau. Cette séparation des deux phases est une opération connue en soi.

L'étape b) consiste à éliminer l'eau et l'alkylamine de cette phase organique. Il est avantageux de procéder par distillation. On peut opérer sous vide ou jusqu'à quelques bars pourvu qu'on ne dépasse pas la température à laquelle la phase organique commence à se dégrader, ou faire des produits colorés ou des produits de décomposition. Cette température est habituellement inférieure à 180 °C. Avantageusement on opère entre 130 et 170 °C. On ne sortirait pas du cadre de l'invention en opérant à plus haute température, mais on risque de dégrader les produits alors qu'il est plus simple d'opérer à températures plus basse.

La durée de l'opération est sans importance, elle est fixée par les conditions pratiques liées à l'appareillage et aux quantités d'eau et l'alkylamine à éliminer.

Cette étape b), de même que toutes les autres étapes de la présente invention, peut être effectuée en continu ou en discontinu. Quand on a éliminé toute l'eau et l'alkylamine, on obtient un reste organique contenant essentiellement l'amide recherchée. On effectue l'étape c) par tout moyen connu de séparation. On utilise avantageusement une distillation ou une ou plusieurs évaporations flash ou une évaporation en film, ou en couche mince et un opérant sous vide.

Dans les exemples suivants le test de stabilité thermique consiste à maintenir l'arylsulfonylalkylamide pendant 3 heures à 250 °C sous azote, la coloraltion en fin de test doit être inférieure à 250 Hazen. Le produit est alors utilisable comme plastifiant.

Sauf indication contraire, on opère dans un réacteur en verre muni d'une agitation, d'une gaine thermométrique, d'un injecteur pour balayage par l'azote, d'un réfrigérant ascendant et d'un refroidissement par bain d'eau froide ou de saumure. Pendant les distillations (étapes b) et c)) on a maintenu une couverture d'azote sur les produites.

EXEMPLE 1

a) On coule en 1 heure 30 minutes 3 moles de benzène sulfochlorure ($C_6H_5SO_2Cl$) dans un mélange contenant 3,051 moles de soude en solution aqueuse à 19,37 % en poids et 3,3 moles de n-butylamine ($CH_3CH_2CH_2CH_2NH_2$). La température du réacteur est maintenu à 20 °C. Puis on porte cette température entre 60 et 65 °C pendant 2 heures. Après décantation on obtient 675 g d'une phase organique contenant 3 X 0,9959 moles de N,n-butylbenzènesulfonamide ($C_6H_5SO_2NHCH_2CH_2CH_2CH_3$) (BBSA).

b) On distille cette phase organique pour éliminer l'eau et la n-butylamine en maintenant la température de pied de 20 à 145 °C sous un vide de 740 à 10 mmHg pendant 1 heure.

c) On distille sous vide (0,5 mmHg) le reste obtenu et on recueille ainsi 96 % du BBSA contenu dans la phase organique en fin de l'étape a). Le test thermique montre une coloration de 50 Hazen.

EXEMPLE 2

On opère comme dans l'exemple 1 sauf que pendant la coulée du benzènesulfochlorure la température est maintenue à 50 °C. Les résultats sont identiques à l'exemple 1.

EXEMPLE 3

a) On coule 0,6 mole de benzènesulfochlorure dans 3,3 moles de n-butylamine en maintenant le réacteur à 50 °C. On coule ensuite simultanément 3,051 moles de soude sous forme de solution aqueuse à 19,37 % en poids et 2,4 moles de benzènesulfochlorure en 1 heure et 30 minutes, le réacteur étant maintenu à 50 °C. On ajoute 7 g d'eau pour rincer l'ampoule de coulage de la soude. On porte ensuite le réacteur pendant 2 heures entre 60 et 65 °C.

Après décantation on obtient 669 g d'une phase organique contenant 3 X 0,9939 moles de BBSA.

b) On distille comme dans l'exemple 1.

c) On distille sous vide de 0,5 mmHg le reste obtenu et on recueille ainsi 95 % du BBSA contenu dans la phase organique en fin de l'étape a). Le test thermique montre une coloration de 50 Hazen.

EXEMPLE 4

On opère comme dans l'exemple 2 mais en utilisant un réacteur en acier inoxydable dont le fonds est en nuance 304 L et le reste en nuance 316 L. On obtient des résultats identiques.

EXEMPLE 5

a) On coule en 30 minutes 3 moles de benzène sulfochlorure dans 3,3 moles de n-butylamine, la température du réacteur est maintenue à 50 °C. Puis on coule en 1 heure et 30 minutes 3,15 moles de soude en solution aqueuse à 19,91 %.

On rince par 13,5 g d'eau l'ampoule de coulage de la soude. On porte le réacteur entre 60 et 70 °C pendant 2 heures. Après décantation on obtient 671,8 g d'une phase organique contenant 3 moles de BBSA.

b) On distille l'eau et l'amine comme dans l'exemple 1. On constate qu'on a perdu 6,1 % de la massage engagée en b) pendant cette distillation.

c) On distille sous vide (0,5 mmHg) le reste obtenu et on recueille ainsi 92 % du BBSA contenu dans la phase organique en fin d'étape a). La test thermique montre une coloration de 175 Hazen.

EXEMPLE 6

a) On opère comme dans l'exemple 3 sauf que le réacteur est maintenu à 20 °C au lieu de 50 °C pendant les deux opérations de coulage des réactifs. Après décantation on obtient 671,8 g d'une phase organique contenant 3 X 0,998

moles de BBSA.

b) On distille l'eau et l'amine comme dans l'exemple 1. On constate qu'on a perdu 6,07 % de la masse pendant cette distillation.

c) On distille sous vide (0,5 mmHg) le reste obtenu et on recueille 3 fractions correspondantes aux têtes, coeurs et queues de distillation (les % sont en poids)

F1 = 4,9 %
F2 = 84,2 %
F3 = 7,1 %

Il reste dans le ballon 3,8 % (% de la masse engagée dans l'étape c)).

Le test thermique sur F2 dans une coloration inférieure à 50 hazen et 100 Hazen sur F1 + F2 + F3.

EXEMPLE 7

On opère comme dans l'exemple 2 sauf qu'on utilise 3,75 moles de n-butylamine. On obtient 702,6 g d'une phase organique contenant 3 X 0,9924 moles de BBSA. On obtient des résultats identiques.

EXEMPLE 8

On opère comme dans l'exemple 2 sauf qu'on utilise 3,15 moles de n-butylamine. On obtient 659,8 g d'une phase organique contenant 3 X 0,9915 moles de BBSA. On obtient des résultats identiques.

**Revendications**

1. Procédé de synthèse d' arylsulfonylalkylamide de formule :

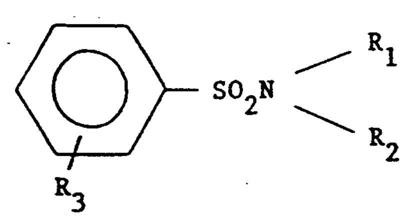

dans laquelle $R_1$ représente un atome d'hydrogène ou un groupe alkyle ayant de 1 à 10 atomes de carbone, $R_2$ représente un groupe alkyle ayant de 1 à 10 atomes de carbone et $R_3$ représente un ou plusieurs substituants identiques ou différents choisis dans le groupe constitué par les halogènes et les alkyles ayant jusqu'à 5 atomes de carbone, par réaction d'un arylsulfohalogénure et d'une alkylamine caractérisé en ce que :

a) on met en contact l'arylsulfohalogénure avec un excès d'alkylamine et une solution aqueuse d'un agent alcalin, la quantité d'agent alcalin étant en excès par rapport à l'arylsulfohalogénure engagé,

b) on élimine l'eau et l'alkylamine de la phase organique obtenue en a) par distillation,

c) du reste obtenu en b) on sépare l'arylsulfonylalkylamide.

2. Procédé selon la revendication 1, caractérisé en ce que l'excès molaire d'alkylamine par rapport à la stoechiométrie est compris entre 5 et 15 %.

3. Procédé selon la revendication 1 ou 2, caractérisé en ce que l'agent alcalin est de la soude.

4. Procédé selon l'une des revendications 1 à 3, caractérisé en ce que l'excès molaire d'agent alcalin est de préférence compris entre 1 et 5 %.

5. Procédé selon l'une des revendications 1 à 5 caractérisé en ce que l' arylsulfonylalkylamide est la N.n-butylbenzène sulfonamide :

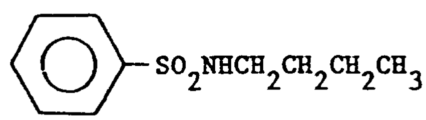

**Claims**

1. Process for the synthesis of arylsulphonylalkylamide of formula:

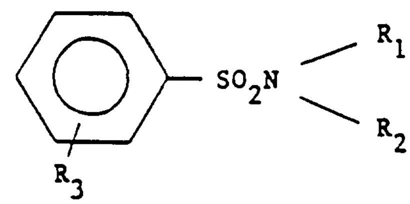

in which $R_1$ represents a hydrogen atom or an alkyl group having 1 to 10 carbon atoms, $R_2$ represents an alkyl group having 1 to 10 carbon atoms and $R_3$ represents one or more identical or different substituents selected from the group consisting of halogens and alkyls having up to 5 carbon atoms, by reaction of an arylsulphonyl halide and an alkylamine, characterized in that:

a) the arylsulphonyl halide is placed in contact with an excess of alkylamine and an aqueous solution of an alkaline agent, the

quantity of alkaline agent being in excess relative to the arylsulphonyl halide involved,

b) water and the alkylamine are eliminated from the organic phase obtained in a), by distillation,

c) the arylsulphonylalkylamide is separated from the residue obtained in b).

**2.** Process according to Claim 1, characterized in that the molar excess of alkylamine relative to the stoichiometry is between 5 and 15 %.

**3.** Process according to Claim 1 or 2, characterized in that the alkaline agent is sodium hydroxide.

**4.** Process according to one of Claims 1 to 3, characterized in that the molar excess of alkaline agent is preferably between 1 and 5 %.

**5.** Process according to one of Claims 1 to 5, characterized in that the arylsulphonylalkylamide is N-(n-butyl)benzenesulphonamide:

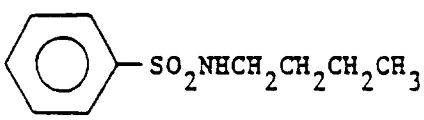

**Patentansprüche**

**1.** Verfahren zur Herstellung von Arylsulfonylalkylamid der allgemeinen Formel

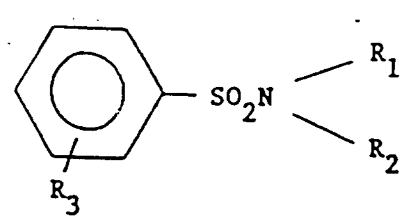

in der $R_1$ ein Wasserstoffatom oder eine Alkylgruppe mit 1 bis 10 Kohlenstoffatomen ist, $R_2$ eine Alkylgruppe mit 1 bis 10 Kohlenstoffatomen ist und $R_3$ eine oder mehrere gleiche oder unterschiedliche Substituenten betrifft, die ausgewählt sind aus der Gruppe bestehend aus Halogenen und Alkyl mit bis zu 5 Kohlenstoffatomen, durch Reaktion eines Arylsulfohalogenids und eines Alkylamins, dadurch gekennzeichnet, daß man

a) ein Arylsulfohalogenid mit einem Überschuß eines Alkylamins und einer wäßrigen Lösung eines alkalischen Mittels in Berührung bringt, wobei die Menge des alkalischen Mittels in Bezug auf das Arylsulfohalogenid im Überschuß eingesetzt wird,

b) das Wasser und das Alkylamin der organischen Phase, die man in a) erhält durch Destillation entfernt, und

c) aus dem in b) erhaltenen Rückstand das Alkylsulfonylalkylamid abtrennt.

**2.** Verfahren nach Anspruch 1, dadurch gekennzeichnet, daß der molare Überschuß des Alkylamins in Bezug auf die Stöchiometrie zwischen 5 und 15 % beträgt.

**3.** Verfahren nach Anspruch 1 oder 2, dadurch gekennzeichnet, daß das alkalische Mittel Ätznatron ist.

**4.** Verfahren nach einem der Ansprüche 1 bis 3, dadurch gekennzeichnet, daß der molare Überschuß an alkalischem Mittel vorzugsweise zwischen 1 und 5 beträgt.

**5.** Verfahren nach einem der Ansprüche 1 bis 4, dadurch gekennzeichnet, daß das Alkylsulfonylalkylamid das folgende N-n-Butylbenzolsulfonamid ist: